# EUROPÄISCHE PATENTANMELDUNG

(11) **EP 4 279 116 A1**
(43) Veröffentlichungstag der Anmeldung: **22.11.2023**
(21) Anmeldenummer: 22174262.0
(22) Anmeldetag: 19.05.2022
(51) Int. Cl.: A61M 27/00, A61M 60/882

(54) **IMPLANTIERBARE PUMPVORRICHTUNG ZUM PUMPEN EINER KÖRPERFLÜSSIGKEIT**

(71) Anmelder: B. Braun Miethke GmbH & Co. Kg, 14469 Potsdam (DE)
(72) Erfinder: Böse, Gordon, 10317 Berlin (DE); Loudermilk, Brandon, 14469 Potsdam (DE); Miethke, Christoph, 14469 Potsdam (DE)
(74) Vertreter: Patentanwälte Ruff, Wilhelm, Beier, Dauster & Partner mbB

(57) **Zusammenfassung**

Implantierbare Pumpvorrichtung (1) zum Pumpen einer Körperflüssigkeit. Ein Kolben (2) ist entlang einer Hubachse (H) hubbeweglich. Ein zwischen einem Kolbenboden (3) und einer Wandung (4) eingeschlossenes Pumpvolumen (V) bildet einen Abschnitt eines zwischen einem Einlass (E) und einem Auslass (A) erstreckten Flüssigkeitspfads. Die Hubbewegung des Kolbens (2) bewirkt eine Veränderung des Pumpvolumens (V) und folglich eine Pumpförderung der Körperflüssigkeit zwischen dem Einlass und dem Auslass. Der Kolben (2) weist einen formstabilen Scheibenabschnitt (5) und einen elastischen Ringkragenabschnitt (6) auf, der radial von dem Scheibenabschnitt (5) abragt, an seinem Innenumfang fest und fluiddicht mit einem Außenumfang des Scheibenabschnitts verbunden ist und an seinem Außenumfang fest und fluiddicht mit der Wandung verbunden ist, wodurch der formstabile Scheibenabschnitt mittels des elastischen Ringkragenabschnitts relativ zu der Wandung hubbeweglich an derselben abgestützt und fluiddicht mit derselben verbunden ist.

## Beschreibung

Die Erfindung betrifft eine implantierbare Pumpvorrichtung zum Pumpen einer Körperflüssigkeit, aufweisend einen entlang einer Hubachse hubbeweglichen Kolben mit einem Kolbenboden, eine dem Kolbenboden entlang der Hubachse gegenüberliegende Wandung, und aufweisend ein zwischen dem Kolbenboden und der Wandung eingeschlossenes Pumpvolumen, welches einen Abschnitt eines zwischen einem Einlass und einem Auslass erstreckten Flüssigkeitspfads bildet, wobei eine Hubbewegung des Kolbens eine Veränderung des Pumpvolumens und folglich eine Pumpförderung der Körperflüssigkeit zwischen dem Einlass und dem Auslass bewirkt.

Eine derartige implantierbare Pumpvorrichtung ist beispielsweise aus der US 9,731,101 B2 bekannt. Die bekannte implantierbare Pumpvorrichtung ist zum Abpumpen von Cerebrospinalflüssigkeit (CSF) aus den Ventrikeln des Gehirns in einen anderen zur Aufnahme der Flüssigkeit geeigneten Hohlraum des Körpers eines Patienten eingerichtet. Zu diesem Zweck wird die bekannte implantierbare Pumpvorrichtung beispielsweise in das Peritoneum und/oder Atrium implantiert und mittels hierfür geeigneter Katheter einlassseitig mit den Ventrikeln und auslassseitig mit dem besagten Hohlraum fluidleitend verbunden. Die bekannte implantierbare Pumpvorrichtung arbeitet nach dem Verdrängerprinzip und kann insbesondere als Peristaltikpumpe, Schraubenpumpe, Membranpumpe oder Kolbenpumpe gestaltet sein. Kolbenpumpen weisen üblicherweise einen beweglichen Kolben, eine Wandung und ein zwischen dem Kolbenboden des Kolbens und der Wandung eingeschlossenes Pumpvolumen auf. Der Kolben ist relativ zu der Wandung linear hubbeweglich. Die Hubbewegung des Kolbens führt bekanntermaßen zu einer Veränderung des Pumpvolumens, welche mit einer Pumpförderung zwischen dem Einlass und dem Auslass einhergeht. Bei üblichen Kolbenpumpen ist die Wandung als Zylinder gestaltet. Bei der Hubbewegung gleitet der Kolben reibungsbehaftet im Inneren des Zylinders, genauer: entlang dessen Zylinderinnenwandung. Zur Abdichtung zwischen Kolben und Zylinderinnenwandung werden üblicherweise gleitbeweglich an der Zylinderinnenwandung anliegende Kolbendichtringe verwendet.

Aufgabe der Erfindung ist es, eine implantierbare Pumpvorrichtung der eingangs genannten Art bereitzustellen, die Vorteile gegenüber dem Stand der Technik bietet.

Diese Aufgabe wird dadurch gelöst, dass der Kolben einen formstabilen Scheibenabschnitt, dessen Unterseite den Kolbenboden bildet, und einen elastischen Ringkragenabschnitt aufweist, wobei der Ringkragenabschnitt radial von dem Scheibenabschnitt abragt, an seinem Innenumfang fest und fluiddicht mit einem Außenumfang des Scheibenabschnitts verbunden ist und an seinem Außenumfang fest und fluiddicht mit der Wandung verbunden ist, wodurch der formstabile Scheibenabschnitt mittels des elastischen Ringkragenabschnitts relativ zu der Wandung hubbeweglich an derselben abgestützt und fluiddicht mit derselben verbunden ist. Durch die erfindungsgemäße Lösung werden ein einfacher Aufbau und eine besonders robuste Funktion erreicht. Damit einhergehend kann die Lebensdauer der implantierbaren Pumpvorrichtung erhöht und deren Ausfallrisiko gesenkt werden. Dies führt zu einer verbesserten Patientensicherheit. Überdies erlaubt der einfache Aufbau eine kostenreduzierte Herstellung sowie eine vereinfachte Montage. Die vorgenannten Vorteile werden durch die erfindungsgemäße Gestaltung des Kolbens und dessen Zusammenwirken mit der Wandung erreicht. Der erfindungsgemäße Aufbau des Kolbens sieht zum einen den formstabilen Scheibenabschnitt und zum anderen den elastischen Ringkragenabschnitt vor. Der Scheibenabschnitt fungiert in erster Linie als Verdrängerelement der nach dem Verdrängerprinzip arbeitenden implantierbaren Pumpvorrichtung. Insbesondere zu diesem Zweck ist der Scheibenabschnitt formstabil. Die Formstabilität erlaubt die Übertragung der zum Pumpen der Körperflüssigkeit notwendigen Kräfte. Der Ringkragenabschnitt dient in erster Linie einer fluiddichten Abdichtung und hubbeweglichen Abstützung des Scheibenabschnitts an der Wandung. Dem Ringkragenabschnitt kommt mithin eine besonders vorteilhafte Mehrfachfunktion zu. Dies trägt maßgeblich zum vereinfachten Aufbau und der robusten Funktion der implantierbaren Pumpvorrichtung bei. Zur Abstützung des Scheibenabschnitts an der Wandung ist der Ringkragenabschnitt zum einen fest mit dem Scheibenabschnitt und zum anderen fest mit der Wandung verbunden. Um trotz der jeweils festen Verbindung die erforderliche Hubbewegung des Kolbens zu ermöglichen, ist der Ringkragenabschnitt elastisch ausgebildet. Folglich wird der Ringkragenabschnitt während der Hubbewegung des Kolbens elastisch deformiert. Infolge der festen Verbindung des Ringkragenabschnitts an seinem Innenumfang einerseits und seinem Außenumfang andererseits findet an den besagten Stellen keine Relativbewegung zu dem Scheibenabschnitt bzw. der Wandung statt. Hierdurch werden Reibungsverluste vermieden. Zur Abdichtung des zwischen der Unterseite des Scheibenabschnitts und der Wandung eingeschlossenen, veränderlichen Pumpvolumens ist die vorbeschriebene feste Verbindung zwischen dem Innenumfang des Ringkragenabschnitts und dem Außenumfang des Scheibenabschnitts einerseits und dem Außenumfang des Ringkragenabschnitts und der Wandung andererseits zudem fluiddicht ausgebildet. Hierdurch kann insbesondere auf gesonderte Dichtelemente verzichtet werden. Die festen und fluiddichten Verbindungen können jeweils als stoffschlüssige, form- und/oder kraftschlüssige Verbindung oder mittels einer einstückig zusammenhängenden Fertigung ausgebildet sein. Die formstabilen Eigenschaften des Scheibenabschnitts und die elastischen und/oder formnachgiebigen Eigenschaften des Ringkragenabschnitts werden jeweils über eine entsprechende Dimensionierung der Wanddicken und/oder Werkstoffwahl erreicht. Vorzugsweise ist der Scheibenabschnitt im Vergleich zu dem Ringkragenabschnitt dickwandig. Vorzugsweise ist der Ringkragenabschnitt im Vergleich zu dem Scheibenabschnitt dünnwandig. Vorzugsweise beträgt der Unterschied zwischen den Wanddicken wenigstens eine Größenordnung, besonders bevorzugt wenigstens zwei Größenordnungen. Der Flüssigkeitspfad der implantierbaren Pumpvorrichtung weist einends den Einlass und andernends den Auslass auf. Das Pumpvolumen bildet einen Abschnitt des Flüssigkeitspfades und ist in Förderrichtung der Körperflüssigkeit zwischen dem Einlass und dem Auslass angeordnet. Der Einlass und der Auslass sind über das Pumpvolumen fluidisch miteinander verbunden. Bei einer Ausgestaltung sind Steuerelemente zur Steuerung der Pumpförderung in dem Flüssigkeitspfad angeordnet, die vorzugsweise als Rückschlagventile gestaltet sind. Bei einer weiteren Ausgestaltung weist die implantierbare Pumpvorrichtung keine solchen Steuerelemente auf. Stattdessen sind entsprechende Steuerelemente beispielsweise abseits der implantierbaren Pumpvorrichtung dem Einlass vor- und/oder dem Auslass nachgelagert, vorzugsweise in einem mit der implantierbaren Pumpvorrichtung verbindbaren Fluidleitungssystem oder dergleichen. Bei einer Ausgestaltung weist die implantierbare Pumpvorrichtung zum Antreiben der Hubbewegung einen mit dem Kolben wirkverbundenen Aktor auf. Der Aktor ist vorzugsweise kraft- und/oder bewegungsübertragend mit dem Kolben, insbesondere dem Scheibenelement, wirkverbunden. Die Wirkverbindung kann mechanisch und/oder fluidisch ausgebildet sein. Prinzipiell denkbar ist zudem eine magnetische Wirkverbindung. Bei einer weiteren Ausgestaltung weist die implantierbare Pumpvorrichtung keinen solchen Aktor auf, wobei der Aktor stattdessen beispielsweise als Bestandteil einer separaten Antriebsvorrichtung mit der implantierbaren Pumpvorrichtung verbindbar sein kann.

Die erfindungsgemäße implantierbare Pumpvorrichtung eignet sich besonders vorteilhaft zum Pumpen von Körperflüssigkeiten, insbesondere von Cerebrospinalflüssigkeit. Selbstverständlich ist die erfindungsgemäße Lösung gleichermaßen zum Pumpen medizinischer Flüssigkeiten geeignet.

In Ausgestaltung der Erfindung sind der Scheibenabschnitt und der Ringkragenabschnitt einstückig miteinander zusammenhängend ausgebildet. Infolge der einstückigen Ausbildung hängt der Ringkragenabschnitt an seinem Innenumfang fest und fluiddicht mit dem Außenumfang des Scheibenabschnitts zusammen und umgekehrt. Folglich kann auf eine gesonderte Fügeverbindung verzichtet werden. Dies führt zu einem weiter vereinfachten Aufbau und in einer nochmals verbesserten Robustheit. Der Kolben kann bei dieser Ausgestaltung der Erfindung beispielsweise als Drehteil, Gussteil oder Umformteil gefertigt sein. Alternativ ist eine additive Fertigung denkbar.

In weiterer Ausgestaltung der Erfindung sind der Scheibenabschnitt und der Ringkragenabschnitt jeweils aus Metall, vorzugsweise aus Titan, gefertigt. Die Erfinder haben erkannt, dass hierdurch besondere Vorteile erreicht werden können. Zum einen führt die Fertigung aus Metall, vorzugsweise aus Titan, zu biokompatiblen Eigenschaften. Zum anderen ergeben sich fertigungstechnische Vorteile. Dies gilt sowohl für eine mehrteilige als auch für eine einstückig zusammenhängende Fertigung von Scheiben- und Ringkragenabschnitt. Vorzugsweise ist auch die Wandung aus Metall, bevorzugt aus Titan, gefertigt. In Kombination mit der metallischen Fertigung des Ringkragenabschnitts ergeben sich insbesondere fertigungstechnische Vorteile. Beispielsweise kann hierdurch die Verbindung zwischen dem Außenumfang des Ringkragenabschnitts und der Wandung mit einfachen Mitteln zuverlässig fest und fluiddicht ausgebildet werden.

In weiterer Ausgestaltung der Erfindung ist der Außenumfang des Ringkragenabschnitts mittels einer stoffschlüssigen Fügeverbindung fest und fluiddicht mit der Wandung zusammengefügt. Die stoffschlüssige Fügeverbindung ist bei einer Ausgestaltung eine Klebeverbindung. Bei einer weiteren Ausgestaltung ist eine Schweißverbindung vorgesehen. Durch diese Ausgestaltung der Erfindung ergeben sich weitere fertigungstechnische Vorteile. Überdies wird der Aufbau der implantierbaren Pumpvorrichtung weiter vereinfacht, da auf gesonderte Verbindungsmittel zur Verbindung des Außenumfangs des Ringkragenabschnitts mit der Wandung verzichtet werden kann.

In weiterer Ausgestaltung der Erfindung ist die stoffschlüssige Fügeverbindung eine, vorzugsweise ohne einen Schweißzusatz gefertigte, Schweißverbindung. In diesem Fall sind der Ringkragenabschnitt und die Wandung vorzugsweise jeweils aus Metall oder schweißbarem Kunststoff gefertigt. Im Vergleich zu beispielsweise einer Klebeverbindung bietet die Schweißverbindung verbesserte biokompatible Eigenschaften. Dies insbesondere dann, wenn die Schweißverbindung ohne einen Schweißzusatz gefertigt wird. In diesem Fall ist die Schweißverbindung beispielsweise eine Laser-, Reibschweiß- oder eine Widerstandsschweißverbindung.

In weiterer Ausgestaltung der Erfindung ist eine Wanddicke und/oder eine Formgebung des Ringkragenabschnitts derart ausgebildet, dass eine hubbedingte elastische Deformation des Ringkragenabschnitts eine der Hubbewegung entgegengesetzte Federkraft bewirkt, wobei die Federkraft eine vollständige Rückstellung des Kolbens bewirkt oder wenigstens unterstützt. Demnach fungiert der Ringkragenabschnitt bei dieser Ausgestaltung zusätzlich als eine Art Federelement zur Rückstellung des Kolbens. Hierdurch kann auf gesonderte Bauelemente zur Rückstellung des Kolbens verzichtet werden. Wenigstens können solche Bauteile weniger stark dimensioniert werden. Mit anderen Worten ausgedrückt, fungiert der Ringkragenabschnitt bei dieser Ausgestaltung vorzugsweise als eine Art Tellerfeder.

In weiterer Ausgestaltung der Erfindung weist der Ringkragenabschnitt eine ebene Formgebung auf. Folglich ist der Ringkragenabschnitt in radialer Richtung und/oder in Umfangsrichtung eben erstreckt. Eine solch ebene Gestaltung unterstützt eine einfache Fertigung. Zudem wird die Herstellung der festen und fluiddichten Verbindung zwischen dem Außenumfang des Ringkragenabschnitts und der Wandung durch die ebene Formgebung erleichtert.

In weiterer Ausgestaltung der Erfindung weist der Ringkragenabschnitt eine gewellte Formgebung auf. Die Wellenform ist in radialer Richtung und/oder in Umfangsrichtung des Ringkragenabschnitts ausgebildet. Vorzugsweise weist der Ringkragenabschnitt mehrere Radialwellen auf. Mit anderen Worten ausgedrückt, ist der Ringkragenabschnitt über seiner radialen Erstreckung mit mehreren Wellen versehen, welche in Umfangsrichtung durchgängig erstreckt sind. Die Erfinder haben erkannt, dass die gewellte Formgebung insbesondere eine anforderungsgerechte elastische Deformation des Ringkragenabschnitts unterstützt. Dies unterstützt eine leichtgängige und dennoch hinreichend abgestützte und/oder geführte Auf- und Abbewegung des mit dem Ringkragenabschnitt verbundenen Scheibenabschnitts.

In weiterer Ausgestaltung der Erfindung weist die Wandung einen weiteren elastischen Ringkragenabschnitt auf, dessen Außenumfang fest und fluiddicht mit dem Außenumfang des elastischen Ringkragenabschnitts des Kolbens verbunden ist. Der weitere Ringkragenabschnitt verbessert das Deformationsverhalten des Ringkragenabschnitts des Kolbens. Das verbesserte Deformationsverhalten geht mit einer besonders leichtgängigen Hubbeweglichkeit des Scheibenabschnitts einher. Dies unterstützt einen besonders energieeffizienten Betrieb der implantierbaren Pumpvorrichtung. Vorzugsweise ragt der weitere Ringkragenabschnitt radial von einem dem Scheibenabschnitt gegenüberliegenden Mittenabschnitt der Wandung nach außen ab. Bei dieser Ausgestaltung der Erfindung ist das Pumpvolumen vorzugsweise zwischen der Unterseite des Scheibenabschnitts und dem besagten Mittenabschnitt eingeschlossen. Der Ringkragenabschnitt des Scheibenelements und der weitere Ringkragenabschnitt der Wandung liegen an ihren Außenumfängen aufeinander auf und sind dort fest und fluiddicht miteinander verbunden. Hierfür kommt wiederum eine stoff-, form- und/oder kraftschlüssige Verbindung infrage. Bevorzugt ist eine Schweißverbindung.

In weiterer Ausgestaltung der Erfindung sind der Ringkragenabschnitt des Kolbens und der weitere Ringkragenabschnitt der Wandung in Bezug auf eine radiale Mittellängsebene des Pumpvolumens spiegelsymmetrisch angeordnet und/oder ausgebildet. Die besagte Spiegelsymmetrie kann zum einen fertigungstechnische Vorteile bieten. Zum anderen ergibt sich ein besonders vorteilhaftes elastisches Deformationsverhalten der beiden Ringkragenabschnitte und damit eine besonders anforderungsgerechte Hubbeweglichkeit des Scheibenabschnitts.

In weiterer Ausgestaltung der Erfindung weist der Flüssigkeitspfad ein erstes Rückschlagventil, welches zwischen dem Einlass und dem Pumpvolumen angeordnet ist, und ein zweites Rückschlagventil, welches zwischen dem Pumpvolumen und dem Auslass angeordnet ist und entgegengesetzt zu dem ersten Rückschlagventil öffnet und schließt, auf. Das erste Rückschlagventil kann auch als Einlassventil bezeichnet werden. Das zweite Rückschlagventil kann auch als Auslassventil bezeichnet werden. Die beiden Rückschlagventile ermöglichen bauartbedingt einen besonders einfachen Aufbau der implantierbaren Pumpvorrichtung. Zudem wird eine besonders robuste und damit gegen Störungen unanfällige Steuerung der Pumpförderung der Körperflüssigkeit ermöglicht. Bei einer weiteren Ausgestaltung sind anstelle von Rückschlagventilen elektronische Steuerventile oder dergleichen vorgesehen.

In weiterer Ausgestaltung der Erfindung sind das erste Rückschlagventil und/oder das zweite Rückschlagventil in die Wandung integriert. Hierdurch ergeben sich unterschiedliche konstruktive Vorteile. Zum einen kann im Vergleich zu einer Integration in den Kolben, die grundsätzlich denkbar ist, eine Reduzierung der oszillierenden Masse erreicht werden. Zum anderen kann vorhandener Bauraum besser genutzt werden. Dies ermöglicht eine besonders kompakte Bauform der implantierbaren Pumpvorrichtung.

Die der Erfindung zugrunde liegende Aufgabe wird zudem dadurch gelöst, dass ein zwischen dem Einlass und dem Pumpvolumen erstreckter Abschnitt des Flüssigkeitspfads an eine Kolbenrückseite des Kolbens, welche dem Kolbenboden entlang der Hubachse gegenüberliegt, angrenzt, wodurch die Kolbenrückseite mit einem einlassseitigen Druck der Körperflüssigkeit druckbeaufschlagt ist. Durch die erfindungsgemäße Lösung wird mit überraschend einfachen Mitteln eine Kompensation von Druckschwankungen ermöglicht. Denn bei aus dem Stand der Technik bekannten Lösungen ist die Kolbenrückseite in üblicher Weise gegenüber einem vorherrschenden Umgebungsdruck gekapselt. Folglich wirken sich Schwankungen des Umgebungsdrucks über die Körperflüssigkeit des Patienten und von dort lediglich auf den Kolbenboden aus. Dies kann bei aus dem Stand der Technik bekannten implantierbaren Pumpvorrichtungen zu einem erhöhten Kraftbedarf für die Hubbewegung des Kolbens führen. Damit einhergehend ergibt sich ein erhöhter Energieverbrauch, der aus unterschiedlichen Gründen nachteilig ist. Bei der erfindungsgemäßen Lösung wirkt der einlassseitige Druck der Körperflüssigkeit - und damit mittelbar der Umgebungsdruck - auf die Kolbenrückseite. Folglich muss zum Pumpen der Körperflüssigkeit lediglich der zwischen dem Pumpvolumen und dem Auslass vorherrschende Differenzdruck überwunden werden. Dies führt zu einer relativ leichtgängigen Hubbewegung und ermöglicht einen besonders energieeffizienten Betrieb der implantierbaren Pumpvorrichtung. Sofern die implantierbare Pumpvorrichtung gemäß einer der vorhergehenden Ausgestaltungen ausgeführt ist, ist die Kolbenrückseite eine der Unterseite entlang der Hubachse gegenüberliegende Oberseite des formstabilen Scheibenabschnitts. Der an die Kolbenrückseite und/oder die Oberseite des Scheibenabschnitts angrenzende Abschnitt des Flüssigkeitspfads kann auch als Kompensationskammer bezeichnet werden. Im Betrieb der implantierbaren Pumpvorrichtung strömt die Körperflüssigkeit ausgehend von dem Einlass durch die Kompensationskammer, von dort in das Pumpvolumen und wird ausgehend von diesem weiter in Richtung Auslass gefördert. Mit anderen Worten ausgedrückt, ist die Kompensationskammer im Betrieb der implantierbaren Pumpvorrichtung stets von der Körperflüssigkeit durchströmt. Hierdurch werden stehende Flüssigkeit und die damit einhergehenden Nachteile vermieden. Insbesondere wird Agglomerationen an der Kolbenrückseite und einer resultierenden Keimbildung oder sonstigen gesundheitsschädlichen Phänomenen entgegengewirkt.

In weiterer Ausgestaltung der Erfindung weist die implantierbare Pumpvorrichtung ein Gehäuse mit einem ersten Innenraum, in welchem der Kolben, die Wandung und der Flüssigkeitspfad angeordnet sind, und einen zweiten Innenraum, welcher gegenüber einer Umgebung und dem ersten Innenraum fluiddicht abgedichtet und zur Aufnahme weiterer Komponenten der Pumpvorrichtung eingerichtet ist, auf. Bei den weiteren Komponenten kann es sich beispielsweise um einen Aktor zum Antreiben der Hubbewegung des Kolbens und/oder eine Steuereinrichtung zum Steuern des Aktors handeln.

Die Erfindung betrifft zudem eine implantierbare Vorrichtung mit einer Pumpvorrichtung nach einem der vorhergehenden Ansprüche. Die implantierbare Vorrichtung ist bei einer Ausgestaltung eine Dialysevorrichtung zur Verwendung bei einer cerebralen Mikrodialyse, wobei die Pumpvorrichtung zum Pumpen von Cerebrospinalflüssigkeit eingerichtet ist. Bei einer weiteren Ausgestaltung dient die implantierbare Vorrichtung einer Schmerztherapie und/oder einer Behandlung von Spastiken.

Weitere Merkmale und Vorteile der Erfindung ergeben sich aus den Ansprüchen sowie aus der nachfolgenden Beschreibung bevorzugter Ausführungsbeispiele der Erfindung, die anhand der Zeichnungen dargestellt sind.
- Fig. 1: zeigt eine schematisch stark vereinfachte Schnittdarstellung einer Ausführungsform einer erfindungsgemäßen implantierbaren Vorrichtung, welche mit einer Ausführungsform einer erfindungsgemäßen implantierbaren Pumpvorrichtung versehen ist,
- Fig. 2: die implantierbare Pumpvorrichtung nach Fig. 1 in einer perspektivischen, geschnittenen Explosionsdarstellung,
- Fig. 3: in schematisch stark vereinfachter Schnittdarstellung einen Kolben, eine Wandung und ein zwischen diesen Bauteilen eingeschlossenes Pumpvolumen der implantierbaren Pumpvorrichtung nach den Fig. 1 und 2,
- Fig. 4: eine Variante der Anordnung nach Fig. 3 und
- Fig. 5: eine weitere Variante der Anordnung nach Fig. 3.

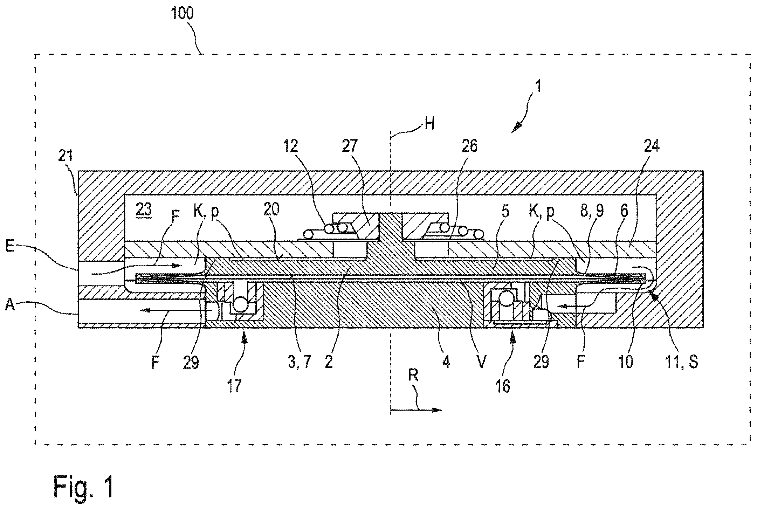

Gemäß Fig. 1 ist eine in den Körper eines Patienten implantierbare Pumpvorrichtung 1 zum Pumpen einer Körperflüssigkeit vorgesehen.

Bei der in Fig. 1 gezeigten Ausführungsform ist die implantierbare Pumpvorrichtung 1 Bestandteil einer schematisch vereinfacht dargestellten implantierbaren Vorrichtung 100. Die implantierbare Vorrichtung 100 ist vorliegend zur Verwendung bei einer cerebralen Mikrodialyse vorgesehen. Insoweit dient die implantierbare Pumpvorrichtung 1 vorliegend zum Pumpen von Cerebrospinalflüssigkeit. Alternativ kann die implantierbare Vorrichtung 100 beispielsweise bei einer Schmerztherapie und/oder zur Behandlung von Spastiken verwendet werden.

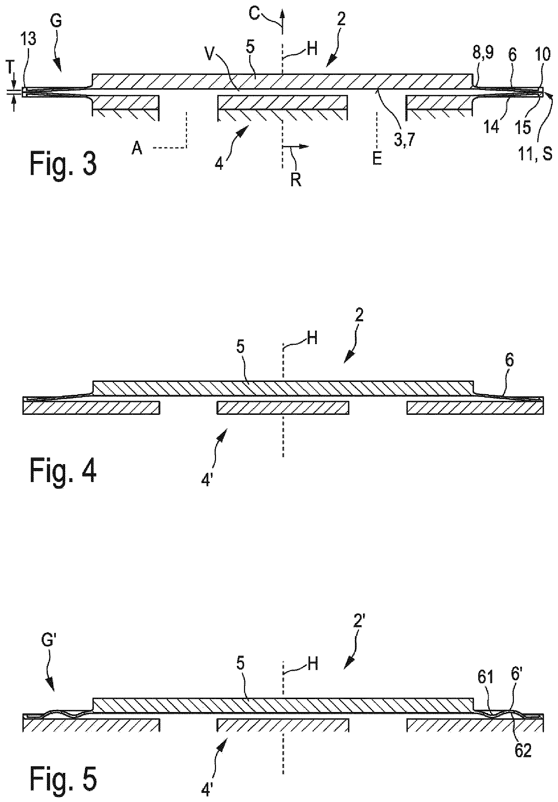

Die implantierbare Pumpvorrichtung 1 (nachfolgend abgekürzt: Pumpvorrichtung) weist einen entlang einer Hubachse H hubbeweglichen Kolben 2 mit einem Kolbenboden 3, eine dem Kolbenboden 3 entlang der Hubachse H gegenüberliegende Wandung 4 und ein zwischen dem Kolbenboden 3 und der Wandung 4 eingeschlossenes Pumpvolumen V auf (siehe auch Fig. 3).Das Pumpvolumen V bildet einen Abschnitt eines zwischen einem Einlass E und einem Auslass A der Pumpvorrichtung 1 erstreckten Flüssigkeitspfads F. Eine lineare Hubbewegung des Kolbens 2 entlang der Hubachse H bewirkt eine Veränderung des Pumpvolumens V und folglich eine Pumpförderung der Körperflüssigkeit zwischen dem Einlass E und dem Auslass A. Bei einer - in Bezug auf die Zeichenebene der Fig. 1 - Abwärtsbewegung des Kolbens 2 wird das Pumpvolumen V verringert. Bei einer Aufwärtsbewegung des Kolbens 2 wird das Pumpvolumen V vergrößert. Die besagte Aufwärtsbewegung bewirkt ein Ansaugen der Körperflüssigkeit in das Pumpvolumen V. Die besagte Abwärtsbewegung bewirkt ein Ausstoßen der Körperflüssigkeit aus dem Pumpvolumen V.

Zur Steuerung der vorbeschriebenen Pumpförderung entlang des Flüssigkeitspfads F weist die Pumpvorrichtung 1 bei der gezeigten Ausführungsform noch näher beschriebene Steuerelemente auf.

Der Kolben 2 weist einen formstabilen Scheibenabschnitt 5 und einen elastischen Ringkragenabschnitt 6 auf. Eine der Wandung 4 entlang der Hubachse H zugewandte Unterseite 7 des Scheibenabschnitts 5 bildet den Kolbenboden 3 aus. Der elastische Ringkragenabschnitt 6 ragt in Radialrichtung R nach außen von dem Scheibenabschnitt 5 ab. Der Ringkragenabschnitt 6 ist in Umfangsrichtung des Scheibenabschnitts 5 durchgängig umlaufend. Der Ringkragenabschnitt 6 weist einen Innenumfang 8 und einen Außenumfang 10 auf. Der Innenumfang 8 kann auch als Ringinnenrand oder innenliegender Ringrand bezeichnet werden. Der Außenumfang 10 kann auch als Ringaußenrand oder außenliegender Ringrand bezeichnet werden. Der Innenumfang 8 ist fest und fluiddicht mit einem Außenumfang 9 des Scheibenabschnitts 5 verbunden. Der Außenumfang 9 des Scheibenabschnitts 5 kann auch als Scheibenaußenrand oder außenliegender Scheibenrand bezeichnet werden. Der Außenumfang 10 des Ringkragenabschnitts 6 ist fest und fluiddicht mit der Wandung 4 verbunden. Hierdurch ist der formstabile Scheibenabschnitt 5 mittels des elastischen Ringkragenabschnitts 6 relativ zu der Wandung 4 zum einen entlang der Hubachse H linear hubbeweglich (mittelbar) an der Wandung 4 abgestützt. Zum anderen ist der formstabile Scheibenabschnitt 5 mittels des Ringkragenabschnitts 6 (mittelbar) mit der Wandung 4 fluiddicht verbunden.

Bei der Pumpförderung der Körperflüssigkeit bewegt sich der formstabile Scheibenabschnitt 5 linear entlang der Hubachse H auf und ab. Aufgrund seiner formstabilen Ausgestaltung erfährt der Scheibenabschnitt 5 hierbei keine oder jedenfalls keine nennenswerte elastische Deformation. Dies gewährleistet insbesondere, dass zur Pumpförderung der Körperflüssigkeit notwendige Pumpkräfte anforderungsgerecht in den Scheibenabschnitt 5 eingeleitet und von diesem auf das Pumpvolumen V übertragen werden können. Im Unterschied zu dem formstabilen Scheibenabschnitt 5 erfährt der Ringkragenabschnitt 6 bei der Hubbewegung eine elastische Deformation. Bei einer Abwärtsbewegung des Kolbens 2 und damit des Scheibenabschnitts 5 wird der Ringkragenabschnitt 6 an seinem Innenumfang 8 entlang der Hubachse H in Richtung der Wandung 4 elastisch deformiert. Der Außenumfang 10 bleibt hierbei relativ zu der Wandung 4 unbeweglich. Die elastische Deformierbarkeit des Ringkragenabschnitts 6 gewährleistet, dass der Scheibenabschnitt 5 sich entlang der Hubachse H linear auf- und abbewegen kann. Die fluiddichte Verbindung sowohl an dem Innenumfang 8 als auch an dem Außenumfang 10 gewährleistet eine zuverlässige fluiddichte Abdichtung zwischen Kolben 2 einerseits und Wandung 4 andererseits. Mit anderen Worten ausgedrückt, fungiert der Ringkragenabschnitt 6 zum einen als eine Art Lager- und/oder Stützelement und zum anderen als eine Art Dichtelement.

Um eine anforderungsgerechte elastische Deformierbarkeit des Ringkragenabschnitts 6 zu erreichen, weist dieser bei der gezeigten Ausführungsform im Vergleich zu dem Scheibenabschnitt 5 eine geringe, noch näher bezeichnete Wanddicke auf. Mit anderen Worten ausgedrückt, ist der Scheibenabschnitt 5 im Vergleich zu dem Ringkragenabschnitt 6 dickwandig gestaltet. Umgekehrt hierzu ist der Ringkragenabschnitt 6 im Vergleich zu dem Scheibenabschnitt 5 dünnwandig gestaltet.

Bei der gezeigten Ausführungsform weist der Scheibenabschnitt 5 eine - in Bezug auf die Hubachse H - flache kreiszylindrische Gestalt auf. Der Außenumfang 9 des Scheibenabschnitts 5 ist somit kreisförmig bzw. kreisrund. Der radial nach außen von dem Scheibenabschnitt 5 abragende Ringkragenabschnitt 6 weist bei der gezeigten Ausführungsform eine kreisringförmige Gestalt auf. Eine solch kreisförmige Gestaltung des Scheibenabschnitts 5 einerseits und des Ringkragenabschnitts 6 ist nicht zwingend erforderlich. Bei einer zeichnerisch nicht dargestellten Ausführungsform sind der Scheibenabschnitt 5 und der Ringkragenabschnitt 6 stattdessen jeweils oval gestaltet.

Bei der gezeigten Ausführungsform sind der Scheibenabschnitt 5 und der Ringkragenabschnitt 6 einstückig miteinander zusammenhängend ausgebildet. Folglich sind der Scheibenabschnitt 5 und der Ringkragenabschnitt 6 unterschiedliche Abschnitte ein- und desselben Bauteils. Durch die miteinander einstückig zusammenhängende Ausbildung kann auf eine gesonderte stoff-, kraft- und/oder formschlüssige Fügeverbindung zwischen dem Innenumfang 8 des Ringkragenabschnitts 6 und des Außenumfangs 9 des Scheibenabschnitts 5 verzichtet werden. Dies vereinfacht den Aufbau und die Herstellung. Zudem kann die erforderliche mechanische und fluiddichte Anbindung zwischen dem Innenumfang 8 und dem Außenumfang 9 besonders robust und zuverlässig ausgebildet werden.

Die besagte einstückige Ausbildung ist zwar besonders vorteilhaft, aber zur Umsetzung der vorliegenden Erfindung nicht zwingend erforderlich. Dementsprechend ist bei einer in den Figuren nicht gezeigten Ausführungsform eine mehrteilige Fertigung des Kolbens vorgesehen, wobei der Scheibenabschnitt und der Ringkragenabschnitt als gesonderte Bauteile gefertigt und hiernach an Innen- und Außenumfang mittels einer hierfür geeigneten Fügeverbindung fest und fluiddicht zusammengefügt sind.

Bei der gezeigten Ausführungsform sind der Scheibenabschnitt und der Ringkragenabschnitt aus Metall gefertigt. Im Speziellen ist eine Fertigung aus Titan vorgesehen. Die Fertigung aus Metall, im Speziellen Titan, geht mit einer anforderungsgerechten Biokompatibilität einher.

Bei einer in den Figuren nicht gezeigten Ausführungsform wird anstelle von Metall ein biokompatibler Kunststoff zur Fertigung des Scheibenabschnitts, des Ringkragenabschnitt und/oder des Kolbens verwendet. Biokompatible Kunststoffe sind dem Fachmann bekannt.

Der Außenumfang 10 des Ringkragenabschnitts 6 ist bei der gezeigten Ausführungsform mittels einer stoffschlüssigen Fügeverbindung 11 fest und fluiddicht mit der Wandung 4 zusammengefügt. Die stoffschlüssige Fügeverbindung 11 ist bei unterschiedlichen Ausführungsformen unterschiedlich ausgeführt. Bei der gezeigten Ausführungsform ist die stoffschlüssige Fügeverbindung 11 eine Schweißverbindung S.

Die Schweißverbindung S kann - je nach vorherrschender Werkstoffwahl - für den Kolben 2 und die Wandung 4 eine Metall- oder eine Kunststoffschweißverbindung sein.

Bei der gezeigten Ausführungsform ist auch die Wandung 4 aus Metall, im Speziellen aus Titan, gefertigt. Die Schweißverbindung S ist dementsprechend eine Metallschweißverbindung.

Die Schweißverbindung S ist bei der gezeigten Ausführungsform ohne die Verwendung eines Schweißzusatzes gefertigt. Hierdurch werden weitere Vorteile erreicht. Zum Ausbilden der Schweißverbindung S geeignete Schweißverfahren sind beispielsweise Laserschweißen, Reibschweißen und/oder Widerstandsschweißen.

Weiter in Bezugnahme auf Fig. 3 ist eine Wanddicke T und/oder eine Formgebung G des Ringkragenabschnitts 6 derart ausgebildet, dass eine hubbedingte elastische Deformation des Ringkragenabschnitts 6 eine der Hubbewegung entgegengesetzte Federkraft C bewirkt, wobei die Federkraft C eine vollständige Rückstellung des Kolbens bewirkt oder wenigstens unterstützt. Bei einer Abwärtsbewegung des Kolbens 2 wird der Ringkragenabschnitt 6 elastisch vorgespannt. Die elastische Vorspannung des Ringkragenabschnitts 6 bewirkt die besagte Federkraft C. Diese wirkt entgegengesetzt zu der Abwärtsbewegung des Kolbens 2. Mit anderen Worten: Der elastisch deformierte Ringkragenabschnitt 6 drückt den formstabilen Scheibenabschnitt 5 in seine Ausgangsposition entlang der Hubachse H zurück. Je nach Dimensionierung der Wanddicke T und spezifischer Formgebung G wird die Rückstellung entweder ausschließlich durch die elastische Deformation bzw. die Federkraft C bewirkt oder wenigstens unterstützt. Vorliegend ist letzteres vorgesehen, wobei ein (zusätzliches) Federelement 12 zur Rückstellung des Kolbens vorhanden und auf noch näher beschriebene Weise mit dem Kolben 2 wirkverbunden ist (siehe Fig. 1).

Bei der gezeigten Ausführungsform beträgt die Wanddicke T 0,07 mm. Bei nicht in den Figuren gezeigten Ausführungsformen beträgt die Wanddicke zwischen 0,04 mm und 0,15 mm.

Bei der gezeigten Ausführungsform beträgt eine nicht näher bezeichnete Wanddicke des formstabilen Scheibenabschnitts 1,5 mm. Bei nicht in den Figuren gezeigten Ausführungsformen beträgt die Wanddicke des Scheibenabschnitts zwischen 1,0 mm und 5,0 mm.

Vorliegend weist der elastische Ringkragenabschnitt 6 im Bereich seines Außenumfangs 10 einen entlang der Hubachse H aufgestellten Axialbund 13 auf. Der Axialbund 13 vereinfacht das Ausbilden der Schweißverbindung S. Zudem unterstützt der Axialbund 13 das elastische Deformationsverhalten des Ringkragenabschnitts 6.

Weiter in Bezugnahme auf Fig. 3 ist die Formgebung G des Ringkragenabschnitts 6 vorliegend in radialer Richtung R als auch in Umfangsrichtung eben ausgeführt. Das heißt es sind keinerlei Erhebungen, Einbuchtungen, Wellen oder dergleichen an dem Ringkragenabschnitt 6 vorhanden.

Bei der in den Fig. 1 bis 3 gezeigten Ausführungsform weist die Wandung 4 ebenfalls einen elastische Ringkragenabschnitt 14 auf. Dieser wird nachfolgend als weiterer Ringkragenabschnitt 14 bezeichnet. Der weitere Ringkragenabschnitt 14 ragt in radialer Richtung R nach außen und weist einen Außenumfang 15 auf. Der Außenumfang 15 des weiteren Ringkragenabschnitts 14 ist fest und fluiddicht mit dem Außenumfang 10 des elastischen Ringkragenabschnitts 6 des Kolbens 2 verbunden. Folglich ist die besagte Fügeverbindung 11, genauer: die Schweißverbindung S, zwischen dem Außenumfang 10 des Ringkragenabschnitts 6 des Kolbens 2 und dem Außenumfang 15 des weiteren Ringkragenabschnitts 14 der Wandung 4 ausgebildet. Infolge des weiteren elastischen Ringkragenabschnitts 14 ist die Wandung 4 abschnittsweise, nämlich im Bereich des weiteren Ringkragenabschnitts 14, elastisch nachgiebig. Hierdurch ist der Scheibenabschnitt 5 während seiner Hubbewegung entlang der Hubachse H über eine Art Reihenschaltung von Federn abgestützt. Die Erfinder haben erkannt, dass das Vorhandensein des weiteren Ringkragenabschnitts 14 mit unterschiedlichen Vorteilen einhergeht. Insbesondere wird die anforderungsgerechte Hubbeweglichkeit des Kolbens 2 unterstützt.

Bei der gezeigten Ausführungsform sind der Ringkragenabschnitt 6 des Kolbens 2 und der weitere Ringkragenabschnitt 14 der Wandung 4 in Bezug auf eine radiale Mittellängsebene des Pumpvolumens V spiegelsymmetrisch angeordnet und ausgebildet. Die Wandung 4 mit ihrem weiteren Ringkragenabschnitt 14 kann auch als weiterer Kolben bezeichnet werden. Dies aufgrund der Tatsachse, dass die Wandung mit ihrem weiteren Ringkragenabschnitt 14 vorliegend in weiten Teilen identisch zu dem Kolben 2 gestaltet ist, wobei ein Hauptunterschied in der einerseits relativ zu der Hubachse H beweglichen und andererseits zu derselben - abgesehen von dem weiteren Ringkragenabschnitt 14 - feststehenden Anordnung der Kolben liegt. Die Wandung 4 kann daher auch als feststehende Wandung bezeichnet werden.

Anhand der Fig. 4 und 5 sind Varianten der Anordnung nach Fig. 3 gezeigt. Nachfolgend wird lediglich auf wesentliche Unterschiede der Varianten eingegangen. Im Übrigen wird auf das zuvor Offenbarte verwiesen.

Anhand der Variante nach Fig. 4 wird deutlich, dass der weitere Ringkragenabschnitt 14 gemäß Fig. 3 nicht zwingend erforderlich ist. Dementsprechend weist die dortige Wandung 4' keinen solchen Ringkragenabschnitt auf. Hiervon abgesehen ist der Kolben 2 der Anordnung nach Fig. 4 identisch mit dem Kolben 2 nach Fig. 3.

Die Variante nach Fig. 5 unterscheidet sich durch eine gewellte Formgebung G' des Ringkragenabschnitts 6' von der Variante nach Fig. 4. Die gewellte Formgebung G' des Ringkragenabschnitts 6' geht mit in axialer Richtung erstreckten Einbuchtungen 61 und Erhebungen 62 einher. Die Einbuchtungen 61 und Erhebungen 62 sind in Umfangsrichtung des Ringkragenabschnitts 6 durchgängig längserstreckt. Alternativ kann auch davon gesprochen werden, dass der Ringkragenabschnitt 6' Wellen, genauer: Radialwellen, aufweist. Die Erfinder haben erkannt, dass die gewellte Formgebung G' eine im Vergleich zu der Variante nach Fig. 4 verbesserte Hubbeweglichkeit des Scheibenabschnitts 5 ermöglicht.

Als bereits erwähnte Steuerelemente zur Steuerung der Pumpförderung sind bei der gezeigten Ausführungsform Rückschlagventile 16, 17 vorhanden. Die Rückschlagventile 16, 17 sind in dem Flüssigkeitspfad F angeordnet. Das Rückschlagventil 16 kann auch als erstes Rückschlagventil oder Einlassventil bezeichnet werden. Das Rückschlagventil 17 kann auch als zweites Rückschlagventil oder Auslassventil bezeichnet werden. Das erste Rückschlagventil 16 ist zwischen dem Einlass E und dem Pumpvolumen V in dem Flüssigkeitspfad F angeordnet. Das zweite Rückschlagventil 17 ist zwischen dem Pumpvolumen V und dem Auslass A in dem Flüssigkeitspfad F angeordnet. Die Schließ- bzw. Öffnungsrichtung der beiden Rückschlagventile 16, 17 ist zueinander entgegengesetzt. Das erste Rückschlagventil 16 öffnet bei einer Aufwärtsbewegung des Kolbens 2 und schließt bei einer Abwärtsbewegung desselben. Das zweite Rückschlagventil 17 öffnet bei einer Abwärtsbewegung des Kolbens 2 und schließt bei einer Aufwärtsbewegung desselben.

Bei der gezeigten Ausführungsform sind die beiden Rückschlagventile 16, 17 in die Wandung 4 integriert. Die Wandung 4 weist zu diesem Zweck eine erste Aufnahmeaussparung 18 und eine zweite Aufnahmeaussparung 19 auf. Die Aufnahmeaussparungen 18, 19 sind entlang der Hubachse H in die Wandung 4 eingesenkt und zur Aufnahme jeweils eines der Rückschlagventile 16, 17 eingerichtet. Die erste Aufnahmeaussparung 18 nimmt das erste Rückschlagventil 16 auf. Die zweite Aufnahmeaussparung 19 nimmt das zweite Rückschlagventil 17 auf.

Bei einer in den Figuren nicht gezeigten Ausführungsform ist wenigstens eines der beiden Rückschlagventile in den Kolben 2 integriert. Bei einer weiteren nicht gezeigten Ausführungsform sind beide Rückschlagventile in den Kolben 2 integriert.

Weiter unter Bezugnahme auf Fig. 1 ist gezeigt, dass der Flüssigkeitspfad F bei der gezeigten Ausführungsform abschnittsweise an einer Kolbenrückseite 20 des Kolbens 2 entlanggeführt ist. Mit anderen Worten: Der Flüssigkeitspfad F weist einen Abschnitt K auf (siehe Fig. 1), der unmittelbar an die Kolbenrückseite 20 des Kolbens 2 angrenzt. Der Abschnitt K ist zwischen dem Einlass E und dem Pumpvolumen V angeordnet. Vorliegend ist der Abschnitt K zudem dem ersten Rückschlagventil 16 in Stromrichtung vorgelagert. Bei der Pumpförderung der Körperflüssigkeit fließt diese ausgehend von dem Einlass E entlang des Flüssigkeitspfads F in den Abschnitt K und von diesem weiter über das erste Rückschlagventil 16 in das Pumpvolumen V. Die Kolbenrückseite 20 ist folglich mit einem einlassseitigen Druck p der zu fördernden Körperflüssigkeit druckbeaufschlagt. Diese Druckbeaufschlagung wirkt bei der gezeigten Ausführungsform im Wesentlichen auf eine gesamte Fläche der Kolbenrückseite 20, welche vorliegend im Übrigen auch die dem Abschnitt K zugewandten Außenseiten des Ringkragenabschnitts 6 umfasst. Aufgrund des in dem Abschnitt K vorherrschenden Drucks p wird der Kolben 2 entlang der Hubachse H in Richtung der Wandung 4 kraftbeaufschlagt. Diese Kraftbeaufschlagung bewirkt eine Druckkompensation und ermöglicht einen besonders energieeffizienten Betrieb der Pumpvorrichtung 1, was nachfolgend noch näher erläutert wird.

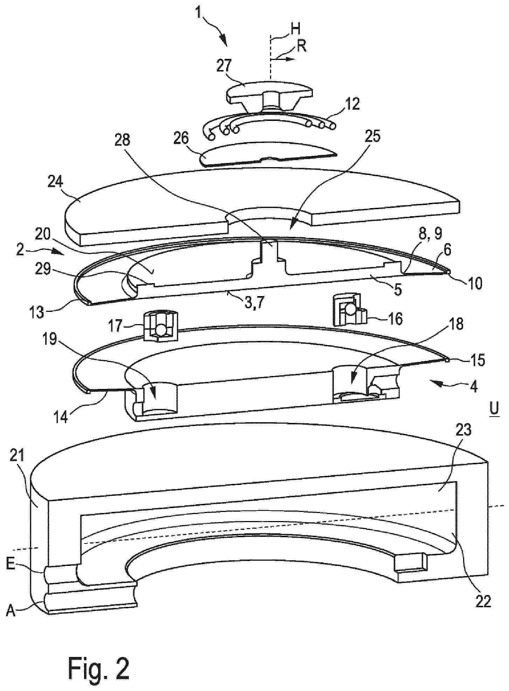

Bei der gezeigten Ausführungsform weist die Pumpvorrichtung ein Gehäuse 21. Das Gehäuse 21 ist in eine untere Hälfte und in eine obere Hälfte untergliedert. Diese Untergliederung ist schematisch anhand Fig. 2 mittels der dortigen strichlierten Linie verdeutlicht. Die besagte Untergliederung unterteilt einen nicht näher bezeichneten Gesamtinnenraum des Gehäuses 21 in einen ersten Innenraum 22 und einen zweiten Innenraum 23. In dem ersten Innenraum 22 sind der Kolben 2, die Wandung 4 und der Flüssigkeitspfad F angeordnet. Der zweite Innenraum 23 ist gegenüber einer Umgebung U und dem ersten Innenraum 22 fluiddicht und/oder druckdicht abgedichtet. Der zweite Innenraum 23 dient der Aufnahme weiterer Komponenten der Pumpvorrichtung 1. Beispielsweise kann in dem zweiten Innenraum 23 ein Aktor zum Antreiben der Pumpbewegung des Kolbens 2 angeordnet sein. Alternativ oder zusätzlich kann eine Steuerelektronik zur Steuerung des Aktors und/oder der Hubbewegung in dem zweiten Innenraum 23 angeordnet sein. Diesbezügliche Details sind zur Umsetzung der vorliegenden Erfindung nicht zwingend erforderlich, so dass von weiteren Ausführungen abgesehen wird.

Zur fluid- und/oder druckdichten Abdichtung des zweiten Innenraums 23 gegenüber der Umgebung U und dem ersten Innenraum 22 ist bei der gezeigten Ausführungsform eine Trennwand 24 vorhanden. Die Trennwand 24 untergliedert den Gesamtinnenraum des Gehäuses 21 in die besagte untere und obere Hälfte bzw. den ersten und zweiten Innenraum 22, 23. Die Trennwand 24 weist vorliegend eine Kreisscheibenform mit einem mittigen Durchlass 25 auf. Der Durchlass 25 dient der axialen Durchführung eines Zapfens 28, der ausgehend von der Kolbenrückseite 20 in axialer Richtung nach oben, d.h. in Richtung des zweiten Innenraums 23, von dem Kolben 2 abragt. Bei der gezeigten Ausführungsform ist der Zapfen 28 einstückig mit den übrigen Abschnitten des Kolbens 2 verbunden. Der Zapfen 28 ist zur kraft- und bewegungsübertragenden Wirkverbindung mit einem Aktor eingerichtet. Mit anderen Worten ausgedrückt, dient der Zapfen 28 der Einleitung von Pumpkräften in den Kolben 2. Die besagten Pumpkräfte werden beispielsweise mit dem vorgenannten und innerhalb des zweiten Innenraums 23 angeordneten Aktor generiert. Der Zapfen 28 durchragt die Durchführung 25 in axialer Richtung und ist mittels einer Dichtscheibe 26 abgedichtet. Die Dichtscheibe 26 ist bei der gezeigten Ausführungsform aus Metall, genauer: Titan, gefertigt. Vorliegend weist die Dichtscheibe 26 membranelastische Eigenschaften auf und kann daher auch als Dichtmembran bezeichnet werden.

In dem anhand Fig. 1 gezeigten Montagezustand ist der Zapfen 28 axial in eine nicht näher bezeichnete Aufnahmebohrung eines Druckstücks 27 eingepasst. Das Druckstück 27 dient der Aufbringung der besagten Pumpkräfte und ist unterseitig entgegen der Abwärtsbewegung des Kolbens 2 mit dem Federelement 12 auf einer nicht näher bezeichneten Oberseite der Trennwand 24 abgestützt. Dabei ist das Federelement 12 vorliegend mittelbar auf der Trennwand, nämlich über die Dichtscheibe 26, abgestützt.

Anhand Fig. 1 wird deutlich, dass der gesamte Flüssigkeitspfad F abseits und getrennt des zweiten Innenraums 23 erstreckt ist. Durch die fluid- und/oder druckdichte Kapselung des zweiten Innenraums 23 ist es nicht zwingend erforderlich, dass die dort angeordneten Komponenten biokompatibel sind. Umgekehrt können die an den Flüssigkeitspfad F angrenzenden Bauteile und/oder Abschnitte vergleichsweise einfach biokompatibel ausgeführt werden. Bei der gezeigten Ausführungsform ist der gesamte Flüssigkeitspfad F bzw. sind sämtliche an den Flüssigkeitspfad F angrenzenden Bauteile und/oder Abschnitte biokompatibel. Diese Biokompatibilität wird vorliegend durch eine entsprechende Werkstoffwahl für den Kolben und die Wandung sowie die Rückschlagventile gewährleistet.

Um zu gewährleisten, dass die Kolbenrückseite 20 auch in einer oberen Endlage des Kolbens 2 - wie sie anhand Fig. 1 gezeigt ist - mit dem einlassseitigen Fluiddruck p druckbeaufschlagt ist, weist der Kolben 2 Abstandsabschnitte 29 auf. Die Abstandsabschnitte 29 liegen in der oberen Endlage an der Trennwand 24, genauer: an deren Unterseite, an. Die Abstandsabschnitte 29 bewirken, dass die Kolbenrückseite 20 nicht unmittelbar an der Trennwand 24 anliegt und der Abschnitt K insoweit zur Druckübertragung auf die Kolbenrückseite 20 offenbleibt.

Bei der gezeigten Ausführungsform bewirkt die Abwärtsbewegung des Kolbens 2 ein Ausstoßen von Körperflüssigkeit aus dem Pumpvolumen V durch den Auslass A und gleichzeitig ein Ansaugen von Körperflüssigkeit durch den Einlass E bis in den Abschnitt K. Bei der Aufwärtsbewegung des Kolbens 2 wird die zunächst bis in den Abschnitt K geförderte Körperflüssigkeit weiter bis in das Pumpvolumen V angesaugt.

Die bereits zuvor erläuterte Druckkompensation ist beispielsweise dann vorteilhaft, wenn sich der Umgebungsdruck (Atmosphärendruck) des Patienten ändert. Eine solche Änderung des Umgebungsdruckes hat naturgemäß auch Auswirkung auf den Druck der Körperflüssigkeit. Deren Druck entspricht dem Umgebungsdruck. In einigen Situationen können beträchtliche Druckänderungen auftreten, beispielsweise bei Flugreisen. In einer Flugzeugkabine kann der Druck bis zu 400 mbar niedriger sein, als am Boden. Der niedrigere Druck liegt folglich einlass- und auslassseitig der Pumpvorrichtung und somit auch in dem Pumpvolumen V an. Der Druck des Innenraums 23 bleibt jedoch konstant, da dieser nach außen hin gekapselt ist. Folglich stellt sich eine Druckdifferenz ein.

Ein Einwirken der Druckdifferenz auf dem gesamten beweglichen Kolben 2 würde in einer Kraft resultieren, welche die Rückstellung des Kolbens 2 in die Ausgangsposition erschweren würde. Um dem entgegenzuwirken können erhöhte Rückstellkräfte über eine Anpassung der Federhärte des Kolbens 2 und/oder des Federelements 12 vorgesehen werden. Auf diese Weise erhöhte Rückstellkräfte sind jedoch unter üblichen Umgebungsdruckbedingungen nachteilig. Denn dann müssten entsprechend erhöhte Pumpkräfte aufgebracht werden, was zu einem größeren Energieverbrauch führen würde.

Kräfte aus vorherrschenden Druckdifferenzen werden vorliegend insbesondere dadurch reduziert, dass die Trennwand 24 eingesetzt wurde, welche das Innere des Gehäuses 21 in einen druckkonstanten Bereich 23 und einen druckangepassten Bereich 22 unterteilt. Die resultierende Kraft aus besagter Druckdifferenz, wirkt nun in erster Linie auf die Fläche der Dichtscheibe 26 und nicht auf dem gesamten Kolben 2. Die benötigten Pumpkräfte sind damit sehr viel weniger abhängig von dem Umgebungsdruck, da dieser Druck an beiden Seiten des Kolbens 2 anliegt.

## Patentansprüche

1. Implantierbare Pumpvorrichtung (1) zum Pumpen einer Körperflüssigkeit, aufweisend
einen entlang einer Hubachse (H) hubbeweglichen Kolben (2, 2') mit einem Kolbenboden (3),
eine dem Kolbenboden (3) entlang der Hubachse (H) gegenüberliegende Wandung (4, 4'), und aufweisend
ein zwischen dem Kolbenboden (3) und der Wandung (4, 4') eingeschlossenes Pumpvolumen (V), welches einen Abschnitt eines zwischen einem Einlass (E) und einem Auslass (A) erstreckten Flüssigkeitspfads (F) bildet,
wobei eine Hubbewegung des Kolbens (2, 2') eine Veränderung des Pumpvolumens (V) und folglich eine Pumpförderung der Körperflüssigkeit zwischen dem Einlass (E) und dem Auslass (A) bewirkt,
**dadurch gekennzeichnet, dass**
der Kolben (2, 2') einen formstabilen Scheibenabschnitt (5), dessen Unterseite (7) den Kolbenboden (3) bildet, und einen elastischen Ringkragenabschnitt (6, 6') aufweist, wobei der elastische Ringkragenabschnitt (6, 6') radial von dem Scheibenabschnitt (5) abragt, an seinem Innenumfang (8) fest und fluiddicht mit einem Außenumfang (9) des Scheibenabschnitts (5) verbunden ist und an seinem Außenumfang (10) fest und fluiddicht mit der Wandung (4) verbunden ist,
wodurch der formstabile Scheibenabschnitt (5) mittels des elastischen Ringkragenabschnitts (6, 6') relativ zu der Wandung (4, 4') hubbeweglich an derselben abgestützt und fluiddicht mit derselben verbunden ist.

2. Implantierbare Pumpvorrichtung (1) nach Anspruch 1, **dadurch gekennzeichnet, dass** der Scheibenabschnitt (5) und der Ringkragenabschnitt (6, 6') einstückig miteinander zusammenhängend ausgebildet sind.

3. Implantierbare Pumpvorrichtung (1) nach Anspruch 1 oder 2, **dadurch gekennzeichnet, dass** der Scheibenabschnitt (5) und der Ringkragenabschnitt (6, 6') jeweils aus Metall, vorzugsweise aus Titan, gefertigt sind.

4. Implantierbare Pumpvorrichtung (1) nach einem der vorhergehenden Ansprüche, **dadurch gekennzeichnet, dass** der Außenumfang (10) des Ringkragenabschnitts (6, 6') mittels einer stoffschlüssigen Fügeverbindung (11) fest und fluiddicht mit der Wandung (4, 4') zusammengefügt ist.

5. Implantierbare Pumpvorrichtung (1) nach Anspruch 4, **dadurch gekennzeichnet, dass** die stoffschlüssige Fügeverbindung (11) eine, vorzugsweise ohne einen Schweißzusatz gefertigte, Schweißverbindung (S) ist.

6. Implantierbare Pumpvorrichtung (1) nach einem der vorhergehenden Ansprüche, **dadurch gekennzeichnet, dass** eine Wanddicke (T) und/oder eine Formgebung (G, G') des Ringkragenabschnitts (6, 6') eine der Hubbewegung entgegengesetzte Federkraft (C) bewirkt, wobei die Federkraft (C) eine vollständige Rückstellung des Kolbens (2, 2') bewirkt oder wenigstens unterstützt.

7. Implantierbare Pumpvorrichtung (1) nach einem der vorhergehenden Ansprüche, **dadurch gekennzeichnet, dass** der Ringkragenabschnitt (6) eine ebene Formgebung (G) aufweist.

8. Implantierbare Pumpvorrichtung (1) nach einem der Ansprüche 1 bis 6, **dadurch gekennzeichnet, dass** der Ringkragenabschnitt (6') eine gewellte Formgebung (G') aufweist.

9. Implantierbare Pumpvorrichtung (1) nach einem der vorhergehenden Ansprüche, **dadurch gekennzeichnet, dass** die Wandung (4) einen weiteren elastischen Ringkragenabschnitt (14) aufweist, dessen Außenumfang (15) fest und fluiddicht mit dem Außenumfang (10) des elastischen Ringkragenabschnitts (6, 6') des Kolbens (2, 2') verbunden ist.

10. Implantierbare Pumpvorrichtung (1) nach Anspruch 9, **dadurch gekennzeichnet, dass** der Ringkragenabschnitt (6, 6') des Kolbens und der weitere Ringkragenabschnitt (14) der Wandung (4) in Bezug auf eine radiale Mittellängsebene des Pumpvolumens (V) spiegelsymmetrisch angeordnet und/oder ausgebildet sind.

11. Implantierbare Pumpvorrichtung (1) nach einem der vorhergehenden Ansprüche, **dadurch gekennzeichnet, dass** der Flüssigkeitspfad (F) ein erstes Rückschlagventil (16), welches zwischen dem Einlass (E) und dem Pumpvolumen (V) angeordnet ist, und ein zweites Rückschlagventil (17), welches zwischen dem Pumpvolumen (V) und dem Auslass (A) angeordnet ist und entgegengesetzt zu dem ersten Rückschlagventil (16) öffnet und schließt, aufweist.

12. Implantierbare Pumpvorrichtung (1) nach Anspruch 11, **dadurch gekennzeichnet, dass** das erste Rückschlagventil (16) und/oder das zweite Rückschlagventil (17) in die Wandung (4, 4') integriert sind.

13. Implantierbare Pumpvorrichtung (1) nach einem der vorhergehenden Ansprüche oder nach dem Oberbegriff des Anspruchs 1, **dadurch gekennzeichnet, dass** ein zwischen dem Einlass (E) und dem Pumpvolumen (V) erstreckter Abschnitt (K) des Flüssigkeitspfads (F) an eine Kolbenrückseite (20) des Kolbens (2, 2'), welche dem Kolbenboden (3) entlang der Hubachse (H) gegenüberliegt, angrenzt, wodurch die Kolbenrückseite (20) mit einem einlassseitigen Druck (p) der Körperflüssigkeit druckbeaufschlagt ist.

14. Implantierbare Pumpvorrichtung (1) nach Anspruch 13, **gekennzeichnet durch** ein Gehäuse (21) mit einem ersten Innenraum (22), in welchem der Kolben (2, 2'), die Wandung (4, 4') und der Flüssigkeitspfad (F) angeordnet sind, und mit einem zweiten Innenraum (23), welcher gegenüber einer Umgebung (U) und dem ersten Innenraum (22) fluiddicht abgedichtet und zur Aufnahme weiterer Komponenten der Pumpvorrichtung (1) eingerichtet ist.

15. Implantierbare Vorrichtung (100) mit einer Pumpvorrichtung (1) nach einem der vorhergehenden Ansprüche.
